(19) Europäisches Patentamt
European Patent Office
Office européen des brevets

(11) Veröffentlichungsnummer: **0 029 546**
**B1**

(12) # EUROPÄISCHE PATENTSCHRIFT

(45) Veröffentlichungstag der Patentschrift: **22.09.82**

(51) Int. Cl.³: **G 03 C 5/54**, G 03 C 7/30, G 03 C 7/32

(21) Anmeldenummer: **80106948.5**

(22) Anmeldetag: **11.11.80**

(54) **Farbphotographisches Aufzeichnungsmaterial mit nicht diffundierenden Elektronendonor-Verbindungen.**

(30) Priorität: **24.11.79 DE 2947425**

(43) Veröffentlichungstag der Anmeldung: **03.06.81 (Patentblatt 81/22)**

(45) Bekanntmachung des Hinweises auf die Patenterteilung: **22.09.82 Patentblatt 82/38**

(84) Benannte Vertragsstaaten: **BE DE FR GB**

(56) Entgegenhaltungen:
**DE A 2 806 196**
**DE A 2 809 716**
**FR A 2 424 566**
**US A 4 197 080**

(73) Patentinhaber: **AGFA-GEVAERT Aktiengesellschaft**
**D-5090 Leverkusen 1 (DE)**

(72) Erfinder: **Odenwälder, Heinrich, Dr.**
**Am Arenzberg 37**
**D-5090 Leverkusen 3 (DE)**
Erfinder: **Vetter, Hans, Dr.**
**Gerstenkamp 19**
**D-5000 Köln 80 (DE)**
Erfinder: **Janssens, Wilhelmus, Dr.**
**Jan-van-Harcourtlaan 35**
**B-3220 Aarschot (BE)**
Erfinder: **Jaeken, Jan, Dr.**
**Wouwstraat 106**
**Hove (BE)**

**EP 0 029 546 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# Farbphotographisches Aufzeichnungsmaterial mit nicht diffudierenden Elektronendonor-Verbindungen

Die Erfindung betrifft ein farbphotographisches Aufzeichnungsmaterial, das nicht diffundierende reduzierbare farbgebenden Verbindungen enthält, die im reduzierten Zustand unter den Bedingungen der alkalischen Entwicklung diffundierende Farbstoffe freisetzen, und das ferner als Reduktionsmittel für die farbgebenden Verbindungen oder sogenannte Elektronendonorverbindungen (im folgenden ED-Verbindungen) reduzierende Sulfonamidoverbindungen enthält.

In der DE-A-2 809 716 sind farbgebende Verbindungen mit einer Elektronen akzeptierenden nukleophilen Vorläufergruppe beschrieben, die im reduzierten Zustand einer intramolekularen nukleophilen Verdrängungsreaktion unterliegen unter Freisetzung eines diffundierenden Farbstoffes. Die Reduktion wird durch sogenannte Elektronendonorverbindungen (ED-Verbindungen) bewiekt, die neben den farbgebenden Verbindungen in den Schichten vorliegen und die bei der Entwicklung bildmäßig oxidiert und damit verbraucht werden. Die verbleibende bildmäßige Verteilung der ED-Verbindungen reagiert mit der farbgebenden Verbindung und löst die bildmäßige Freisetzung diffundierender Farbstoffe aus. Bei den dort beschriebenen ED-Verbindungen handelt es sich beispielsweise um Derivate des Benzisoxazolons, des Hydrochinons, des p-Aminophenols und der Ascorbinsäure. Um die Erzeugung qualitativ hochwertiger Bilder nach dem in DE-A-2 809 716 beschriebenen Verfahren zu ermöglichen, müssen die ED-Verbindungen nicht nur die Farbgebenden Verbindungen reduzieren, sondern sich selbst auch durch belichtetes Silberhalogenid bzw. durch Silberhalogenidentwickleroxidationsprodukte oxidieren lassen. Darüber hinaus müssen die jeweiligen Geschwindigkeiten der Oxidations- bzw. Reduktionsreaktion in optimaler Weise so aufeinander eingestellt sein, daß die ED-Verbindung bei der Entwicklung bereits in bemerkenswertem Umfang oxidiert ist, bevor sie ihrerseits die farbgebende Verbindung zu reduzieren vermag. Die bekannten ED-Verbindungen genügen diesen Anforderungen nicht in jeder Hinsicht, was sich beispielsweise in einer unzureichenden Farbdichte und/oder in einem nicht hinnehmbaren Farbschleier der erzeugten Farübertragungsbilder äußert.

Der Erfindung liegt die Aufgabe zugrunde neue ED-Verbindungen für das Farbdiffusionsübertragungsverfahren auzugeben, die hinsichtlich ihres Reduktionsvermogens und ihrer Oxidierbarkeit den an sie gestellten Forderungen genügen und die hinsichtlich der Qualität der erzeugten Farbbilder den bekannten ED-Verbindungen überlegen sind. Diese Aufgabe wird durch das nachstehend beschriebene farbphotographische Aufzeichnungsmaterial gelöst.

Gegenstand der Erfindung ist ein farbphotographisches Aufzeichnungsmaterial mit mindestens einer Silberhalogenidemulsionsschicht und einer dieser zugeordneten Kombination aus einer nichtdiffundierenden reduzierbaren farbgebenden Verbindung, die im reduzierten Zustand unter den alkalischen Entwicklungsbedingungen einen diffundierenden Farbstoff freizusetzen vermag, und einer Elektronendonorverbindung (ED-Verbindung), die unter den alkalischen Entwicklungsbedingungen die nichtdiffundierende reduzierbare barbgebende Verbindung zu reduzieren vermag, dadurch gekennzeichnet, daß das Material als ED-Verbindung eine Verbindung der folgenden allgemeinen Formel enthält :

$$R^1\,(-L^1=L^2)_n-NH-SO_2-X \qquad (I)$$

worin bedeuten

$L^1$, $L^2$ je eine gegebenenfalls substituierte Methingruppe, die Glied eines wenigstens partiell ungesättigten, vorzugsweise aromatischen carbocyclischen oder heterocyclischen Ringsystems sein kann,

$R^1$ $-OR^2$, $-SR^2$ oder $-NHR^3$,

$R^2$ Wasserstoff oder einen unter den Bedingungen der photographischen Entwicklung hydrolysierbaren Rest, z.B. einen Acylrest,

$R^3$ Wasserstoff, Alkyl, Aryl, Acyl oder einen Rest, der zusammen mit dem in $R^1$ definierten Stickstoffatom und $L^1$ gegebenenfalls unter Einbeziehung von $L^2$ ein Ringsystem mit mindestens einem 5-, 6- oder 7-gliedrigen heterocyclischen Ring vervollständigt,

n 1 oder 2,

X einen beliebigen nicht farbigen organischen Rest.

Der Rest X unterliegt, abgesehen davon, daß er nicht farbig sein soll oder jedenfalls nicht zu einer Anfärbung beitragen soll, keinerlei Beschränkungen. Dies beruht im wesentlichen darauf, daß die Wirksamkeit der ED-Verbindungen bei der erfindungsgemäßen Verwendung weitgehend unbeeinflußt bleibt von der Art des Restes X. Dies wiederum eröffnet die Möglichkeit, daß X in vielfältiger Weise variiert werden kann je nach Anforderung. X kann beispielsweise ein relativ kleiner Molekülrest sein, der nach Abspaltung aus der ED-Verbindung (als $H_2N-SO_2-X$) relativ gute Beweglichkeit in den photographischen Schichten oder im alkalischen Entwicklermedium aufweist, oder ein Rest, der aufgrund seiner Größe nicht mehr beweglich ist. Im allgemeinen werden für X solche Reste in Betracht gezogen, die photographisch inert sind, so daß sich aus der Anwesenheit dieser Reste in den photographischen Schichten keinerlei Beeinträchtigung der photographischen Eigenschaften ergibt. Man kann sich aber auch die Reaktionsweise der erfindungsgemäßen Verbindungen in der Weise zu nutze machen, daß man die ED-Verbindungen mit solchen Resten X ausstattet, die in freigesetzter Form als $H_2N-SO_2-X$

bestimmte Wirkungen in den photographischen Schichten hervorrufen. Die Auswahl eines geeigneten Restes X gestaltet sich demnach entsprechend der gewünschten Funktion. Für X kommen somit beispielsweise nach Belieben aliphatische oder aromatische, gegebenenfalls auch heterocyclische Reste in Frage, wobei die genannten Reste vielfältigster Weise weiter substituiert sein können. Beispiele für X sind etwa Methyl, n-Hexadecyl, Dimethylamino, Phenyl, Naphthyl, p-Dodecylphenyl, p-Tolyl, 3,4-Dichlorphenyl, 4-Dodecyloxyphenyl, 3-Carboxyphenyl, 3-Aminophenyl, 4-N-n-Dodecylcarbamoylaminophenyl.

Falls n den Wert 2 annimmt, befinden sich die Reste $R^1$ und $—NH—SO_2—X$ vorzugsweise in 1,4-Stellung eines Benzolringes, der deswreiteren carbocyclische oder heterocyclische Ringe ankondensiert enthalten kann. In diesem Fall sind die Reste $L^1$ und $L^2$ jeweils zweimal in alternierender Abfolge vorhanden, wobei die beiden durch das gleiche Symbol ($L^1$, $L^2$) dargestellten Methingruppen nicht notwendigerweise identisch sein müssen, was die jeweilige Substitution betrifft. Bevorzugt nimmt n aber den Wert 1 an, so daß man es mit Verbindungen zu tun hat, bei denen $R^1$ und die Gruppe $—NH—SO_2—X$ an verschiedene Kohlenstoffatome einer Vinylengruppe gebunden sind. Letztere kann für sich Bestandteil eines wenigstens partiell ungesättigten, vorzugsweise aromatischen carbocyclischen oder heterocyclischen Ringes sein, der vorzugsweise 5, 6 oder 7 Ringglieder in Gestalt von Kohlenstoff- oder Heteroatomen aufweist, und der weitere Ringe in ankondensierter Form enthalten kann. Derartige Ringe können gegebenenfalls auch den Rest $R^1$ in die Ringbildung mit einbeziehen.

Geeignet sind beispielsweise ED-Verbindungen der forgenden Formel

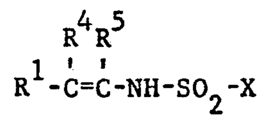

(II)

worin bedeuten :

$R^1$ $—OR^2$, $—SR^2$, $—NHR^3$,

$R^2$ Wasserstoff oder einen unter den Bedingungen der photographischen Entwicklung hydrolysierbaren Rest, z.B. einen Acylrest,

$R^3$ Wasserstoff, Alkyl, Aryl, Acyl oder einen Rest, der zusammen mit $R^4$ oder mit $R^5$ oder mit $R^4$ und $R^5$ ein Ringsystem mit mindestems einem 5-, 6- oder 7-gliedrigen heterocyclischen Ring vervollständigt,

$R^4$ Wasserstoff, Hydroxyl, Alkyl, Aryl, Acyl oder einen heterocyclischen Rest, einschließlich solcher Alkyl-, Aryl-, Acyl- oder heterocyclischer Reste, die zusammen mit wenigstens einem der Reste $R^1$ und $R^5$ ein Ringsystem mit mindestens einem 5-, 6- oder 7-gliedrigen Ring vervollständigen, oder ein Stickstoffatom mit zwei Substituenten, von denen der eine ein Wasserstoffatom ist oder ein Alkyl-, Aryl- oder Acylrest, einschließlich solcher Alkyl-, Aryl- oder Acylreste, die zusammen mit $R^1$ einen 5-, 6- oder 7-gliedrigen Heteroring mit mindestens einem Stickstoffatom vervollständigen, und von denen der andere ein Wasserstoffatom ist oder ein Rest, der zusammen mit $R^5$ einen 5-, 6- oder 7-gliedrigen Heteroring mit mindestens einem Stickstoffatom vervollständigt, wobei die beiden Substituenten am Stickstoffatom nicht gleichzeitig Wasserstoff sein können,

$R^5$ Wasserstoff oder einen Rest, der zusammen mit $R^1$ einen 5-, 6- oder 7-gliedrigen heterocyclischen stickstoffhaltigen Ring oder zusammen mit $R^4$ einen 5-, 6- oder 7-gliedrigen carbocyclischen oder heterocyclischen Ring vervollständigt,

X einen beliebigen nicht farbigen organischen Rest.

Besonders geeignet sind ED-Verbindungen der folgenden Formel (III)

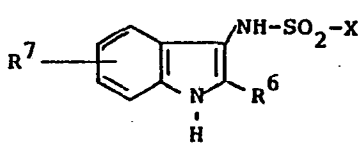

(III)

worin bedeuten

X einen beliebigen nichtfarbigen organischen Rest,

$R^6$ Wasserstoff, Hydroxyl, Alkyl, Aryl, Carbamoyl oder Sulfonylamino ($—NH—SO_2—R$, wobei R = Alkyl, Aryl oder Aralkyl),

$R^7$ Wasserstoff, Alkyl, Aryl, Aralkyl, Halogen, z.B. Cl, $NO_2$, CN, COOH, COOR, Carbamoyl, $SO_3H$, Sulfamoyl, Acylamido, OH, OR, Acyloxy, wobei R = Alkyl, Aryl, Aralkyl. $R^7$ kann auch zwei oder mehrer gleiche oder verschiedene der genannten, von Wasserstoff verschiedene Substituenten bedeuten, wobei zwei derartige Substituenten einen ankondensierten Benzol vervollständigen können.

Bei den erwähnten Alkylresten kann es sich um geradkettige oder verzweigte Alkylreste mit beispielsweise 1 bis 22 C-Atomen handeln, z.B. Methyl, Ethyl, tert.-Butyl, tert.-Amyl, n-Octyl, N-Dodecyl, n-Hexadecyl, n-Octadecyl. Bei den erwähnten Arylresten handelt es sich insbesondere um Phenyl, das gegebenenfalls weiter substituiert ist, z.B. mit Alkyl, Alkoxy, Alkylthio, Alkylsulfonyl, Hydroxyl, Halogen,

Acylamino. Beispielsweise kann eine als $R^6$ vorhandene Phenylgruppe eine Hydroxylgruppe vorzugsweise in p- oder o-Stellung enthanten oder einen Substituenten der vorgenannten Art mit vorzugsweise längerem Alkylrest. Bei den erwähnten Aralkylresten handelt es sich insbesondere um Benzyl. Bei den erwähnten Carbamoyl- oder Sulfamoylresten kann es sich um unsubstituierte Carbamoyl- und Sulfamoylreste handeln oder auch um substituierte, bei denen am Stickstoffatom ein oder zwei Substituenten in Form von Alkyl, Aryl oder Aralkylresten anstelle von Wasserstoffatomen vorhanden sind. Die erwähnten Acylreste, die beispielsweise in den Substituenten $R^2$, $R^3$ und $R^4$ oder in den bei der Definition von $R^6$ und $R^7$ als Substituent erwähnten Gruppen Acylamido und Acyloxy vorhanden sind, leiten sich beispielsweise ab von organischen aliphatischen oder aromatischen Carbon- oder Sulfonsäuren sowie von Carbaminsäuren, Sulfaminsäuren oder Kohlensäurehalbestern, wie Acetyl, Benzoyl, p-Toluolsulfonyl, N-Phenylcarbamoyl, N-n-Octadecylsulfamoyl oder Phenoxycarbonyl.

Beispiele für Ringe, die durch $R^1$ und $R^4$ vervollständigt werden, sind Diazolin- und Diazinonringe und davon abgeleitete benzokondensierte Ringsysteme. Ein Beispiel für Ringe, die durch $R^1$ und $R^5$ vervollständigt werden, ist das Indolringsystem. Beispiele für Ringe, die durch $R^4$ und $R^5$ vervollständigt werden, sind Benzol- und Pyrazolringe sowie das Naphthalinringsystem.

Vorzugsweise ist die Gesamtheit der in einer erfindungsgemäßen ED-Verbindung vorhandenen Substituenten so beschaffen, daß die ED-Verbindung nicht diffundierend in photographische Schichten eingelagert werden kann. Hierzu enthält beispielsweise mindestens einer der vorhanden Substituenten z.B. in ED-Verbindungen der Formel (II) mindestens einer der Reste $R^1$, $R^4$ und $R^5$ oder ein Substituent an einem durch mindestens 2 der genannten Reste vervollständigten Ring, oder auch die Gruppe X einen diffusionsfestmachenden Rest. Eine diffusionsfeste Einlagerung der ED-Verbindungen ist deshalb besonders erwünscht, weil die ED-Verbindungen in einer bestimmten Mengenrelation zu den zugehörigen, nicht diffundierenden, reduzierbaren, farbgebenden Verbindungen eingesetzt werden, die sich möglichst auch während einer längeren Lagerung des photographischen Aufzeichnungsmaterials nicht wesentlich ändern soll.

Als diffusionsfestmachende Reste sind solche Reste anzusehen, die es ermöglichen, die erfindungsgemäßen Verbindungen in den üblicherweise bei photographischen Materialien verwendeten hydrophilen Kolloiden diffusionsfest einzulagern. Hierzu sind vorzugsweise organische Reste geeignet, die im allgemeinen gradkettige oder verzweigte aliphatische Gruppen und gegebenenfalls auch carboxyclische oder heterocyclische aromatische Gruppen mit im allgemeinen 8-20 C-Atomen enthalten. Mit dem übrigen Molekülteil sind diese Reste entweder direkt oder indirekt, z.B. über eine der folgenden Gruppen verbunden : —NHCO—, —$NHSO_2$—, NR— ; wobei R Wasserstoff oder Alkyl bedeutet, —O— oder —S—. Zusätzlich kann der diffusionsfestmachende Rest auch wasserslöslichmachende Gruppen enthalten, wie z.B. Sulfogruppen oder Carboxylgruppen, die auch in anionischer Form vorliegen können. Da die Diffusionseigenschaften von der Molekülgröße der verwendeten Gesamtverbindung abhändigen, genügt es in bestimmten Fällen, z.B. wenn das verwendete Gesamtmolekül groß genug ist, oder wenn die ED-Verbindungen unter Verwendung sogenannter Ölbildner oder hochsiedender Kupplerlösungsmittel in emulgierter Form in die Schichten eingearbeitet werden, als « diffusionsfestmachende Reste » auch kürzerkettige Reste, z.B. Isoamyl- oder tert.-Butylreste, zu verwenden.

Beispiele für erfindungsgemäße ED-Verbindungen sind etwa die folgenden :

1.

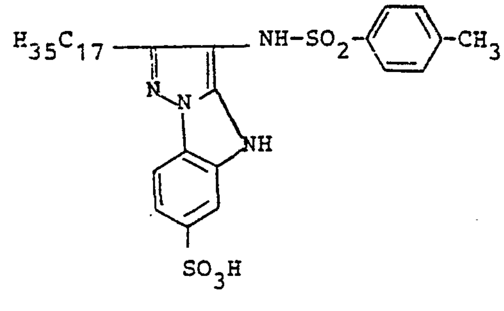

2.

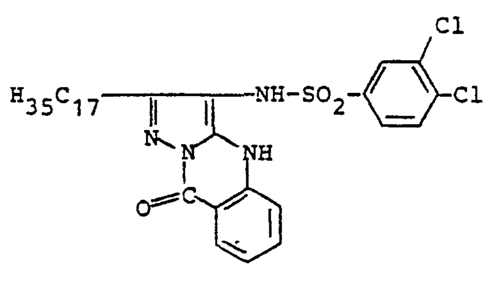

3. 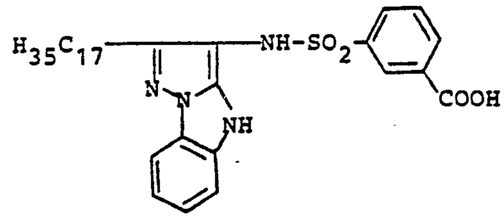

4. 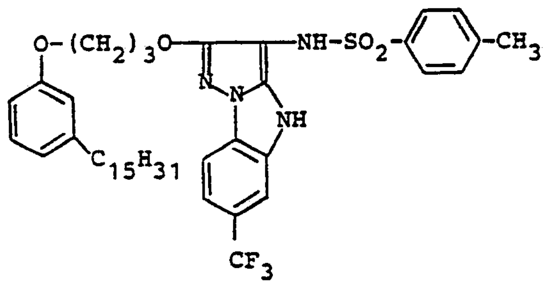

5. 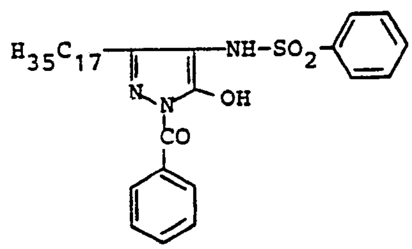

6. 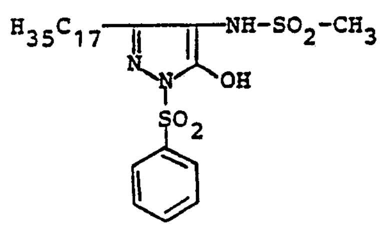

7. 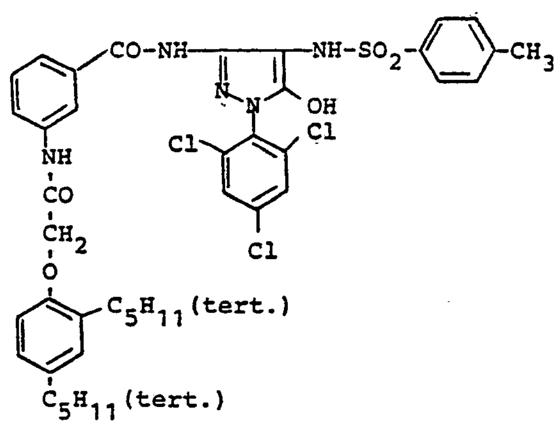

8. 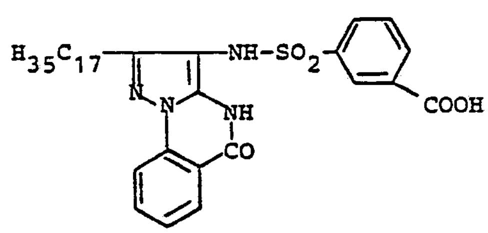

9. 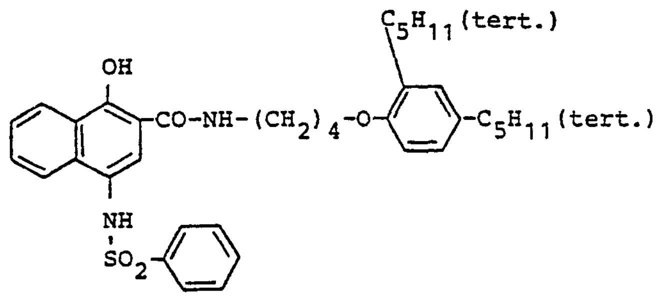

10. 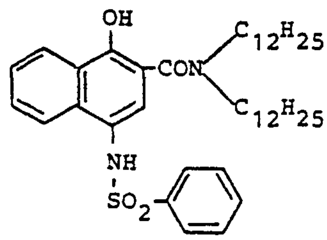

11. 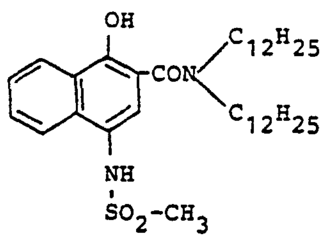

12. 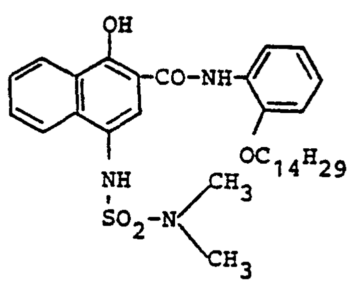

13. 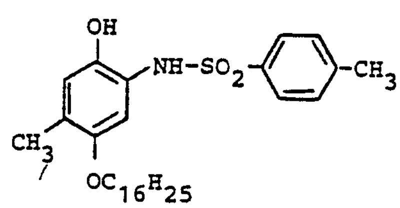

14. 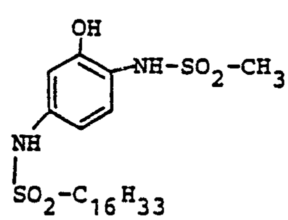

Verbindungen der Formel (III)

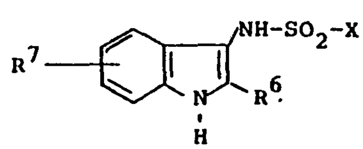

| Nr. | $R^6$ | $R^7$ | X |
|---|---|---|---|
| 15 | $-CONH-(CH_2)_4-O-$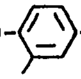$-C_5H_{11}(t)$ $C_5H_{11}(t)$ | $5-OCH_3$ | 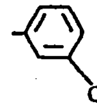 COOH |
| 16 | 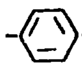$-OC_{16}H_{33}$ | H | 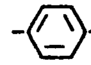$-CH_3$ |
| 17 | 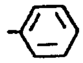 | $5-NH-SO_2-$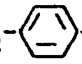$-CH_3$ | $-C_{16}H_{33}$ |
| 18 | 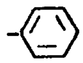 | H | $-C_{16}H_{33}$ |
| 19 | 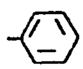 | H | 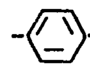$-NHCONHC_{12}H_{25}$ |
| 20 | 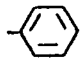 | H | 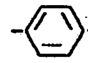$-NHCOC_{11}H_{23}$ |
| 21 | 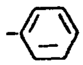 | $5-NHCOC_{11}H_{23}$ | 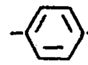$-CH_3$ |
| 22 | 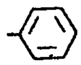 | $5-NHSO_2C_{16}H_{33}$ | 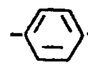$-CH_3$ |
| 23 | 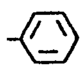 | $5-NHSO_2C_{16}H_{33}$ | $-C_{16}H_{33}$ |
| 24 | 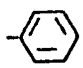 | $5-NHCOC_{11}H_{23}$ | $-CH_3$ |
| 25 | $-CH_3$ | $5-NHCOC_{11}H_{23}$ | $-C_{16}H_{33}$ |
| 26 | 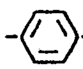$-OC_{16}H_{33}$ | H | 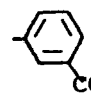 COOH |
| 27 | 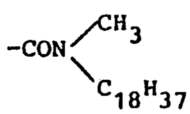 | $5-OCH_3$ | $-C_{16}H_{33}$ |
| 28 | $-CONH-C_{18}H_{37}$ | $5-OCH_3$ | 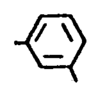 $NH_2$ |

Die Herstellung der erfindungsgemäßen ED-Verbindungen erfolgt nach an sich bekannten Methoden, indem beispielsweise zunächst in bekannter Weise die Verbindungen der allgemeinen Formel IV hergestellt werden

$$R^1(-L^1 = L^2)_n-NH_2 \qquad (IV)$$

wobei $R^1$, $L^1$, $L^2$ und n in der angegebenen Weise definiert sind, und anschließend die Verbindungen der Formel (IV) in ebenfalls bekannter Weise sulfonyliert werden. Verfahren zur Herstellung der Zwischenprodukte der Formel (IV) sind beispielsweise beschrieben in DE-A-2242 762, DE-A-2 406-664, DE-A-2 505 248, DE-A-2 613 005 und DE-A-2 645 656.

Nachstehend ist als Beispiel die Herstellung der Verbindung Nr. 23 beschrieben.

Verbindung Nr. 23

1) 5-Hexadecylsulfonylamino-2-phenyl-indol

20,8 g 5-Amino-2-phenyl-indol werden in 200 ml Pyridin gelöst und mit 32,45 g Hexadecylsulfonylchlorid versetzt. Nach 2 Stunden Rühren bei Raumtemperatur wird auf Zugabe von Wasser ein Niederschlag erhalten, der abgesaugt und mit Wasser gewaschen wird. Der Niederschlag wird in Methanol kalt verrührt, abgesaugt und danach aus Ethanol umkristallisiert unter Zusatz einer gesättigten

wäßrigen Natriumdithionitlösung. Der Niederschlag wird abgesaugt, mit Wasser und wenig Methanol gewaschen und getrocknet. Es werden so 47,4 g der obengenannten Verbindung erhalten.

2) 5-Hexadecylsulfonylamino-3-nitroso-2-phenylindol

42 g der nach 1) erhaltenen Verbindung werden in 400 ml Eisessig angeschlämmt und unter Rühren werden 11,76 g $NaNO_2$ gelöst in 11 ml Wasser tropfenweise zugeben. Es wird 2 Stunden nachgerührt, der Niederschlag danach abgesaugt, in Wasser verrührt, abgesaugt, nochmals in Methanol verrührt, abgesaugt und getrocknet. Es werden so 42,5 g der obengenannten Verbindung erhalten.

3) 3-Amino-5-hexadecylsulfonylamino-2-phenylindol

42,5 g der unter 2) erhaltenen Verbindung werden fein gesiebt in 400 ml Methanol suspendiert und unter Rühren mit 34,8 g Na-dithionit gelöst in 150 ml Wasser versetzt. Die Suspension wird 4 Stunden bei 50 °C gerührt, wobei durch Zugabe von 2 n NaOH pH 9-10 eingestellt wird. Nach Erkalten der Reaktionsmischung wird der Niederschlag abgesaugt und mit Wasser, danach mit Methanol, gewaschen. Nach Trocknen werden 38 g der obengenannten Verbindung vom Schmelzpunkt 182-183 °C erhalten.

4) 3,5-Bis-hexadecylsulfonylamino-2-phenylindol

10,2 g der unter 3) erhaltenen Verbindung in 100 ml Pyridin werden unter Rühren mit 6,6 g Hexadecylsulfochlorid versetzt und das Gemisch 3 Stunden gerührt. Durch Zugabe von Wasser wird ein Niederschlag erhalten, der abgesaugt, mit Wasser gewaschen und danach mit 100 ml Methylenchlorid zu einer Suspension verrührt wird. Nach Zugabe von Methanol wird der Niederschlag abgesaugt und getrocknet. Es werden 14,4 g der obengenannten Verbindung (Verbindung 23) mit dem Schmelpunkt 210-212 °C erhalten.

Die erfindungsgemäßen ED-Verbindungen, sind den bekannten ED-Verbindungen, z.B. Ascorbylpalmitat, insofern überlegen, als sich mit ihnen höhere Maximalfarbdichten und geringere Minimalfarbdichten (Schleier) erzeugen lassen.

Einige der erfindungsgemäßen ED-Verbindungen sind beschrieben in Research Disclosure 17 842, Februar 1979. Sie werden dort als sogenannte « scavenger compounds » bezeichnet, die mit den eingesetzten farbgebenden Verbindungen um das Entwickleroxidationsprodukt konkurieren. Die dort in Betracht gezogenen farbgebenden Verbindungen müssen daher anders als in der vorliegenden Erfindung in einer durch Entwickleroxidationsprodukte oxidierbaren Form vorliegen. Im Gegensatz dazu werden bei der vorliegenden Erfindung farbgebende Verbindungen eines anderen Typs verwendet, nämlich solche, die zunächst einer Reduktion unterworfen werden müssen, bevor sie einen diffundierenden Farbstoff freisetzen. Als Reduktionsmittel dienen erfindungsgemäß die ED-Verbindungen. Es konnte aus der obengenannten Publikation nicht hergeleitet werden, daß die beschriebenen Verbindungen in der Lage sein würden, die hier erfindungsgemäß verwendeten reduzierbaren farbgebenden Verbindungen zu reduzieren und zwar dies in einer gewünschten abgestuften Reaktivität. Erfindungsgemäß soll nämlich die ED-Verbindung zunächst mit den angebotenen Entwickleroxidationsprodukten reagieren, bevor eine nennenswerte Reaktion mit der farbgebenden Verbindung stattfindet. Auf diese Weise wird erreicht, daß die vorhandene ED-Verbindung an den Stellen, wo starke Silberhalogenidentwicklung stattfindet, weitgehend verbraucht wird, während sie an den übrigen Stellen durch Reaktion mit der farbgebenden Verbindung diffundierenden Farbstoff freisetzt. Bei Verwendung von Negativemulsionen wird somit an den belichteten Stellen die ED-Verbindung verbraucht und an den nicht belichteten Stellen diffundierender Farbstoff freigesetzt. Es entsteht ein positives Diffusionsbild.

Als nichtdiffundierende reduzierbare farfgebende Verbindungen, die mit den erfindungsgemäßen ED-Verbindungen in Kombination verwendet können, eignen sich beispielsweise die sogenannten BEND-Verbindungen der DE-A-2 809 716 (BEND = « ballasted electron-accepting nucelophilic displacement »). Diese Verbindungen erfordern zur Farbstoff-Freisetzung eine durch die Reduktion ermöglichte intramolekulare nucleophile Verdrängungsreaktion. Ein anderer Typ von ebenfalls geeigneten nichtdiffundierenden reduzierbaren farbgebenden Verbindungen ist Gegenstand der offengegegten europäischen Patentanmeldung 0 004 399.

Das erfindungsgemäße farbphotographische Aufzeichnungs-material enthält nun bei monochromatischen Verfahren mindestens eine, bei Verfahren zur Herstellung mehrfarbiger Bilder in der Regel mindestens drei bilderzeugende Schichteinheiten, von denen jede mindestens eine lichtempfindliche Silberhalogenidemulsionsschicht und eine diese zugeordnete Kombination aus einer nichtdiffundierenden reduzierbaren farbgebenden Verbindung und einer Elektronendonorverbindung enthält, wobei in mindestens einer Schichteinheit eine erfindungsgemäße ED-Verbindung verwendet wird. In der Regel ist eine der Schichteinheiten überwiegend für blaues Licht empfindlich, eine weitere für grünes Licht und eine dritte für rotes Licht, wobei die zugeordneten farbgebenden Verbindungen jeweils komplementärfarbige Bildfarbstoffe liefern.

Unter « Zuordnung » und « zugeordnet » wird verstanden, daß die gegenseitige Anordnung von Silberhalogenidemulsionen, ED-Verbindung und farbgebender Verbindung von solcher Art ist, daß eine

Wechselwirkung zwischen ihnen möglich ist, die eine bildgemäße Übereinstimmung zwischen dem gebildeten Silberbild und dem Verbrauch an ED-Verbindung einerseits sowie zwischen der nicht verbrauchten ED-Verbindung und der farbgebenden Verbindung andererseits zuläßt, so daß in Übereinstimmung mit dem nicht entwikkelten Silberhalogenid eine bildweise Verteilung von diffundierendem Farbstoff erzeugt wird. Hierzu müssen lichtempfindliches Silberhalogenid, farbgebende Verbindung und ED-Verbindung nicht notwendigerweise in der selben Schicht vorliegen ; sie können auch in zueinander benachbarten Schichten untergebracht sein, die jeweils der gleichen Schichteinheit angehören. Zweckmäßigerweise sind zwischen verschiedenen Schichteinheiten Trennschichten vorhanden, die Verbindungen enthalten können, die mit diffundierenden Entwicklungsprodukten zu reagieren und deren Diffusion aus einer Schichteinheit in eine andere zu unterbinden vermögen. Dies trägt dazu bei, daß die Zuordnung jeweils auf eine Schichteinheit begrenzt bleibt. Derartige Verbindungen sind bekannt, hierfür eignen sich beispielsweise nicht diffundierende Hydrochinonderivate sowie beispielsweise die in der oben erwähnten Publikation Research Disclosure No. 17 842 beschriebenen « scavenger compounds ». Nicht zuletzt können auch die erfindungsgemäßen ED-Verbindungen diese Funktion übernehmen, wenn sie in eine Trennschicht zwischen verschiedenen Schichteinheiten eingelagert werden.

Die Wechselwirkung zwischen dem belichteten Silberhalogenid und der nicht diffundierenden ED-Verbindung wird im allgemeinen durch die oxidierte Form des zur Anwendung gelangenden Silberhalogenidentwicklungsmittels bewerkstelligt. Letzteres wird bei der Entwicklung bildmäßig oxidiert und das Oxidationsprodukt ist seinerseits in der Lage, die ED-Verbindung zu oxidieren und damit der Reaktion mit der farbgebebenden Verbindung zu entziehen. Die Wechselwirkung zwischen der nicht oxidierten ED-Verbindung und der farbgebenden Verbindungwiederum wird durch sogenannte Elektronenübertragungsmittel gefördet. Diese Elektronenübertragungsmittel sind vorzugsweise diffusionsfähig und zu einer reversiblen Aufnahme und Abgabe von Elektronen befähigt. In der Regel sind sie identisch mit den zur Anwendung gelangenden Silberhalogenidentwicklungsmitteln.

Typische geeignete Elektronenübertragungsmittel sind beispielsweise Hydrochinonverbindungen, z.B. Hydrochinon, 2,5-Dichlorhydrochinon und 2-Chlorhydrochinon ; Aminophenolverbindungen wie beispielsweise 2-Aminophenol, N-Methylaminophenol, 3-Methyl-4-aminophenol sowie 3,5-Dibromaminophenol ; Brenzkatechinverbindungen, wie beispielsweise Brenzkatechin, 4-Cyclohexylbrenzkatechin, 3-Methoxybrenzkatechin und 4-(N-Octadecylamino)-brenzkatechin ; Phenylendiaminverbindungen, wie beispielsweise N,N-Diethyl-p-phenylendiamin, 3-Methyl-N,N-diethyl-p-phenylendiamin, 3-Methoxy-N-ethyl-N-hydroxyethyl-p-phenylendiamin sowie N,N,N',N'-Tetramethyl-p-phenylendiamin.

Gemäß einer besonders vorteilhaften Ausgestaltung der Erfindung wird als Elektronenübertragungsmittel eine 3-Pyrazolidonverbindung verwendet, nämlich beispielsweise :

1-Phenyl-3-pyrazolidon, 1-Phenyl-4,4-dimethyl-3-pyrazolidon, 4-Hydroxymethyl-4-methyl-1-phenyl-3-pyrazolidon ; 1-m-Tolyl-3-pyrazolidon, 1-p-Tolyl-3-pyrazolidon, 1-Phenyl-4-methyl-3-pyrazolidon, 1-Phenyl-5-methyl-3-pyrazolidon, 1-Phenyl-4,4-bis-(hydroxymethyl)-3-pyrazolidon, 1,4-Dimethyl-3-pyrazolidon, 4-Methyl-3-pyrazolidon, 4,4-Dimethyl-3-pyrazolidon, 1-(3-Chlorphenyl)-4-methyl-3-pyrazolidon, 1-(4-Chlorphenyl)-4-methyl-3-pyrazolidon, 1-(3-Chlorphenyl)-3-pyrazolidon, 1-(4-Chlorphenyl)-3-pyrazolidon, 1-(4-Tolyl)-4-methyl-3-pyrazolidon, 1-(2-Tolyl)-4-methyl-3-pyrazolidon, 1-(4-Tolyl)-4-hydroxymethyl-4-methyl-3-pyrazolidon, 1-(3-Tolyl)-3-pyrazolidon, 1-(3-Tolyl)-4,4-dimethyl-3-pyrazolidon, 1-(2-Trifluorethyl)-4,4-dimethyl-3-pyrazolidon und 5-Methyl-3-pyrazolidon.

Auch läßt sich eine Kombination von verschiedenen Elektronenübertragungsmitteln verwenden, z.B. eine Kombination, wie sie in US-A-3 039 869 beschrieben ist.

Die Entwicklerverbindungen können in der Entwicklungsflüssigkeit zur Anwendung gebracht werden oder können mindestens teilsweise in irgendeiner Schicht oder Schichten des photographischen Aufzeichnungsmaterials untergebracht werden, z.B. in einer oder mehreren Silberhalogenidemulsionsschichten, Schichten, in denen die Bildfarbstoffe liefernden Verbindungen untergebracht werden, Zwischenschichten, Bildempfangsschichten und dergleichen.

Die im Einzelfalle optimale Elektronenübertragungsverbindung hängt natürlich von der im Einzelfalle verwendeten ED-Verbindung ab, sowie der nicht diffundierenden reduzierbaren farbgebenden Verbindung sowie den Entwicklungsbedingungen des verwendeten Aufzeichnungsmaterials.

Die nichtdiffundierende reduzierbare farbgebende Verbindung wird in einer Schicht in der Regel in einer solchen Menge verwendet, die ausreicht um ein Farbbild mit möglichst hoher maximaler Farbdichte zu erzeugen, beispielsweise in einer Menge von $1\text{-}20.10^{-4}$ Mol/$m^2$. Die erfindungsgemäße ED-Verbindung ist in der Menge an diejenige der farbgebenden Verbindung angepaßt. Sie soll groß genug sein um eine möglichst hohe maximale Farbdichte zu erzielen, d.h. um eine möglichst vollständige Reduktion der farbgebenden Verbindung bewirken zu können. Andererseits soll sie auch nicht wesentlich höher sein als hierzu erforderlich, so daß sie an den belichteten Stellen durch die Entwicklung des belichteten Silberhalogenids möglichst vollständig verbraucht werden kann. Die im Einzelfall günstigsten Mengenverhältnisse zwischen Silberhalogenid, ED-Verbindung und farbgebender Verbindung werden zweckmäßigerweise anhand von Routineversuchen ermittelt. Brauchbare Ergebnisse können beispielsweise erhalten werden, wenn die Menge der ED-Verbindung jeweils in Abhängigkeit von der Menge der farbgebenden Verbindung zwischen $0{,}2.10^{-3}$ und $4.10^{-3}$ Mol/$m^2$ beträgt.

Zur Durchführung des Farbstoffdiffusionsübertragungsverfahrens, wird in der Regel ein lichtempfindliches Element verwendet, das eine oder mehrere Silberhalogenidemulsionsschichteinheiten sowie

die diesen zugeordneten nichtdiffundierenden farbgebenden Verbindungen und ED-Verbindungen enthält, und ein Bildempfangselement, in dem durch die bildmäßige Übertragung diffundierender Farbstoffe das gewünschte Farbbild erzeugt wird. Hierzu ist es erfoederlich, daß zwischen dem lichtempfindlichen Element und dem Bildempfanselement mindestens während eines endlichen Zeitraumes innerhalb der Entwicklungszeit ein fester Kontakt besteht, so daß die in dem lichtempfindlichen Element als Folge der Entwicklung erzeugte bildmäßige Verteilung an diffundierenden Farbstoffen auf das Bildempfangselement übertragen werden kann. Der Kontakt kann hergestellt werden, nachdem die Entwicklung in Gang gesetzt worden ist, oder er kann bereits hergestellt worden sein, bevor die Entwicklung beginnt. Letzteres ist beispielsweise der Fall, falls zur Durchführung des Farbstoffdiffusionsübertragungsverfahrens ein sogenanntes integrales Aufzeichnungsmaterial verwendet wird, in dem das lichtempfindliche Element und das Bildempfangselement eine integrale Einheit bilden, im folgenden auch als Monoblattmaterial bezeichnet, die auch nach Beendigung des Entwicklungsvorganges weiter bestehen bleibt ; d.h., eine Abstrennung des lichtempfindlichen Elementes vom Bildemfangselement ist auch nach erfolgtem Farbübertrag nicht vorgesehen. Eine solche Ausführungsform ist beispielsweise beschrieben in der DE-A-2 019 430.

Das Bildempfangselement kann demnach Bestandteil des farbphotographischen Aufzeichnungsmaterials sein, z.B. in Gestalt einer Bildempfangsschicht, die unterhalb der lichtempfindlichen Silberhalogenidemulsionsschichteinheiten auf einem transparenten Träger angeordnet ist, wobei sich zweckmäßigerweise zwischen der Bildempfangsschicht und den lichtempfindlichen Schichten ein lichtundurchlässige, vorzugsweise lichtreflektierende Bindemittelschicht befindet. Die Bildempfangsschicht, die bei einer anderen Ausgestaltung des Farbübertragungsverfahrens aber auch auf einen separaten Träger angeordnet sein kann (Bildempfangsblatt), enthält in der Regel in bekannter Weise ein basisches Beizmittel für diffundierende anionische (saure) Farbstoffe.

Als Beizmittel für saure Farbstoffe dienen vorzugsweise langkettige quaternäre Ammonium- oder Phosphoniumverbindungen oder tertiäre Sulfoniumverbindungen, z.B. solche, wie sie beschrieben sind in US-A-3 271 147 und US-A-3 271 148. Ferner können auch bestimmte Metallsalze und deren Hydroxide, die mit den sauren Farbstoffen schwerlösliche Verbindungen bilden, verwandt werden. Die Farbstoffbeizmittel sind in der Empfangsschicht in einem der üblichen hydrophilen Bindemittel dispergiert, z.B. in Gelatine, Polyvinylpyrrolidon, ganz oder partiell hydrolysierten Celluloseestern. Selbstverständlich können auch manche Bindemittel als Beizmittel fungieren, z.B. Mischpolymerisate oder Polymerisatgemische von Vinylalkohol und N-Vinylpyrrolidon, wie beispielsweise beschrieben in der DE-B 1 130 284, ferner solche, die Polymerisate von stickstoffhaltigen quaternären Basen darstellen, z.B. Polymerisate von N-Methyl-2-vinylpyridin, wie beispielsweise beschrieben in US 2 484 430. Weitere brauchbare beizende Bindemittel sind beispielsweise Guanylhydrazonderivate von Alkylvinylketonpolymerisaten, wie beschrieben in der US-A-2 882 156 oder Guanylhydrazonderivate von Acylstyrol-polymerisaten, wie beispielsweise beschrieben in der DE-A 2 009 498. Im allgemeinen wird man jedoch auch den zuletzt genannten beizenden Bindemitteln andere Bindemittel, z.B. Gelatine, zusetzen. Weitere polymere Beizmittel sind beschrieben etwa in US-A-3 709 690, DE-A-2 315 304, DE-A-2 445 782, DE-A-2 551 786, DE-A-2 631 521.

Das erfindungsgemäße farbphotographische Aufzeichnungsmaterial kann darüber hinaus noch saure Schichten und sogenannte Brems- oder Verzögerungsschichten enthalten, die zusammen ein sogenanntes Neutralisationsystem bilden. Ein solches Neutralisationssystem kann in bekannter Weise zwischen dem Schichtträger und der darauf angeordneten Bildempfangsschicht angeordnet sein oder an einer anderen Stelle im Schichtverband, z.B. oberhalb der lichtempfindlichen Schichten, d.h. jenseits dieser lichtempfindlichen Schichten, von der Bildempfangsschicht aus betrachtet. Das Neutralisationssystem ist normalerweise so orientiert, daß sich die Brems- oder Verzögerungsschicht zwischen der Säureschicht und der Stelle befindet, an der die alkalische Entwicklungsflüssigkeit oder -paste zur Einwirkung gebracht wird. Solche Säureschichten, Bremsschichten bzw. aus beiden bestehende Neutralisationssysteme sind beispielsweise bekannt aus US-A-2 584 030, US-A-2 983 606, US-A-3 362 819, US-A-3 362 821, DE-A-2 455 762, DE-A-2 601 653, DE-A-2 716 505, DE-A-2 601 653, DE-A-2 716 505, DE-A-2 816 878. Ein solches Neutralisationssystem kann in bekannter Weise auch zwei oder mehrere Bremsschichten enthälten.

Das erfindungsgemäße Aufzeichnungsmaterial kann in einer besonderen Ausgestaltung des weiteren eine oder mehrere für wäßrige Flüssigkeiten durchlässige pigmenthaltige opake Schichten enthalten. Diese können zwei Funktionnen erfüllen. Einerseits kann hierdurch der unerwünschte Zutritt von Licht zu lichtempfindlichen Schichten unterbunden werden und andererseits kann eine solche Pigmentschicht insbesondere, wenn ein helles oder weißes Pigment z.B. $TiO_2$ verwendet werden, für das erzeugte Farbbild einen ästhetisch angenehmen Hintergrund bilden. Integrale farbphotographische Aufzeichnungsmaterialien mit einer solchen Pigmentschicht sind bekannt, z.B. aus US-A-2 543 181 und DE-B-1 924 430. Anstelle einer vorgebildeten opaken Schicht können auch Mittel vorgesehen sein um eine solche Schicht erst im Verlauf des Entwicklungsverfahrens zu erzeugen. Entsprechend den beiden erwähnten Funktionen können derartige Pigmentschichten aus zwei oder mehreren Teilschichten aufgebaut sein, von denen eine beispielsweise ein weißes Pigment und die andere beispielsweise ein dunkles, Licht absorbierendes Pigment, z.B. Ruß, enthält.

Bei einer besonders bevorzugten Ausgestaltung der Erfindung ist das photographische Material ein

integrales farbphotographisches Aufzeichnungsmaterial für die Durchführung des Farbdiffusionsübertragungsverfahrens und weist beispielsweise folgende Schichtelemente auf :

1) eine transparenten Schichtträger
2) eine Bildempfangsschicht
3) eine lichtungdurchlässige Schicht
4) ein lichtempfindliches Element mit mindestens einer lichtempfindlichen Silberhalogenidemulsionsschicht und mindestens einer dieser zugeordneten nichtdiffundierenden, farbgebenden Verbindung sowie einer ED-Verbindung
5) eine Verzögerungsschicht
6) eine saure Polymerschicht
7) einen transparenten Schichtträger

Das Monoblattmaterial kann dabei in der Weise zusammengesetzt werden, daß getrennt voneinander zwei verschiedene Teile hergestellt werden, nämlich der lichtempfindliche Teil (Schichtelemente 1-4) und das Abdeckblatt (Schichtelemente 5-7), die dann schichtseitig aufeinander gelegt und miteinander verbunden werden, gegebenenfalls unter Verwendung von Abstandsstreifen, so daß zwischen den beiden Teilen ein Raum für die Aufnahme einer genau bemessenen Menge einer Entwicklungsflüssigkeit gebildet wird. Die Schichtelemente 5 und 6, die zusammen das Neutralisationssystem bilden, können auch, allerdings in vertauschter Reihenfolge, zwischen dem Schichtträger und der Bildempfangsschicht des lichtempfindlichen Teiles angeordnet sein.

Es können weiterhin Mittel vorgesehen sein, um eine Entwicklungsflüssigkeit zwischen den lichtempfindlichen Teil und das Abdeckblatt einzuführen, z.B. in Form eines seitlich angeordneten, aufspaltbaren Behälters, der bei Einwirkung mechanischer Kräfter seinen Inhalt zwischen zwei benachbarte Schichten des Monoblattmaterials ergießt.

Die Entwicklerzubereitung kann außer dem wäßrigen Alkali noch Entwicklerverbindungen enthalten, die jedoch auf die Art der Farbgebenden Verbindungen abzustimmen sind. Weitere mögliche Bestandteile der Entwicklerzubereitung sind Verdickungsmittel zur Steigerung der Viskosität, z.B. Hydroxyethylcellulose, Silberhalogenidlösungsmittel, z.B. Natriumthiosulfat oder eine der in DE-A-2 126 661 beschriebenen Bissulfonylalkanverbindungen, Trübungsmittel zur Erzeugung von opaken Schichten, z.B. Pigmente von $TiO_2$, ZnO, Bariumstearat oder Kaolin. Alternativ oder zusätzlich können einige dieser Bestandteile auch in eine oder mehrere Schichten des erfindungsgemäßen farbphotographischen Aufzeichnungsmaterials eingelagert sein.

## Beispiel

### Herstellung der Dispergate

1. Blaugrünfarbstoff

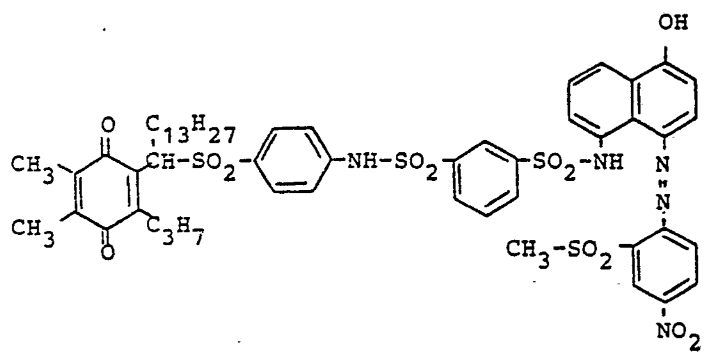

81,5 ml destilliertes Wasser
5 g Gelatine
5 g Blaugrünfarbstoff
3 ml Netzmittel Lomar D (Naphthalinsulfonat-kondensat, Handelsprodukt der Fa. Nopco, USA, Warenzeichen)
5 ml Isopropanol
0,5 g Natriumacetat

Das Gemisch wird in der Sandmühle gemahlen.

2. Magentafarbstoff

15

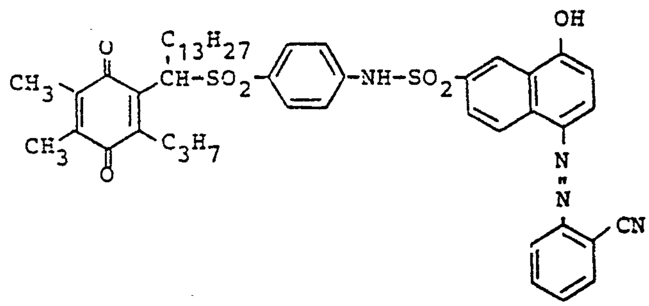

93 ml destilliertes Wasser
1,9 g Gelatine
1,9 g Magentafarbstoff
1,14 ml Netzmittel Lomar D (Warenzeichen)
1,9 ml Isopropanol
0,5 g Natriumacetat

Das Gemisch wird in der Sandmühle gemahlen.
3. 4-Methyl-1-phenyl-3-pyrazolidon

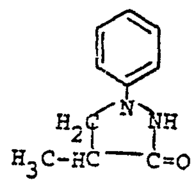

850 ml destilliertes Wasser
40 g Gelatine
100 g 4-Methyl-1-phenyl-3-pyrazolidon
10 ml Netzmittel Lomar D (Warenzichen) 40 %ig

Das Gemisch wird in der Sandmühle gemahlen.
4. Ascorbylpalmitat (ED-Verbindung Vergleich)

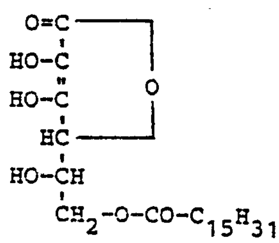

A { 450 ml Ethanol
50 g Ascorbylpalmitat }

B { 1 975 ml destilliertes Wasser
125 g Gelatine }

C { 4,8 g NaOH
95 ml destilliertes Wasser
0,2 ml Octylalkohol }

Lösung A wird unter schnellem Rühren zu einer Mischung der Lösungen B und C eingerührt.
5. ED-Verbindung 16 (erfindungsgemäß)

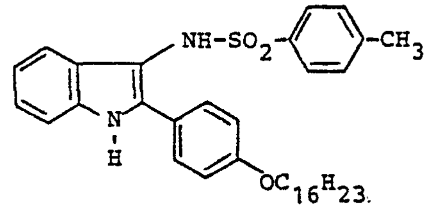

68 ml destilliertes Wasser
4 g Gelatine
4 g ED-Verbindung 16
4 ml N-Methylpyrrolidon

Die ED-Verbindung 16 wird in Ethylacetat und dem N-Methylpyrrolidon aufgelöst, anschließend der aufgelösten Gelatine zugesetzt, worauf das Ethylacetat abgezogen wird.

In gleicher Weise werden auch Dispergate hergestellt von den erfindungsgemäßen ED-Verbindungen 9, 14, 17, 19, 21, 23, 24.

Herstellung der lichtempfindlichen Materialien

Auf einen opaken Schichtträger aus polyethylenkaschiertem Papier wurde eine Silberhalogenidemulsionsschicht mit Farbstoff und ED-Verbindung aufgebracht, wie nachstehend für die Materialien I bis XII angegeben. Darüber wurde wie folgt eine Schutzschicht mit Phenidon vergossen :

938,5 ml destilliertes Wasser
27,5 g Gelatine
24 g Dispergat von 4-Methyl-1-phenyl-3-pyrazolidon
10 ml Netzmittel FT-248 (Handelsprodukt der Fa. Bayer, Leverkusen, Warenzeichen)

Auf diese Weise wurden mehrere lichtempfindliche Materialien hergestellt, wobei für die Silberhalogenidemulsionsschichten folgende Gießlösungen verwendet wurden.

Material I

145 g Silberchloridemulsion mit 86 g $AgNO_3$/kg und einem Gelatine/Silber-Verhältnis von 1,2.
155 ml destilliertes Wasser
54 g Dispergat des Blaugrünfarbstoffes
270 g Dispergat von Ascorbylpalmitat
366 ml destilliertes Wasser
10 ml Netzmittel Hostapon-T 5 %ig (Handelsprodukt der Fa. Hoechst, Warenzeichen)

Silberhalogenidemulsion, Farbstoffdispergat und Dispergat der ED-Verbindung wurden getrennt bei 40 °C aufgeschmolzen. Der aufgeschmolzenen Emulsion wurde zuerst der Farbstoff und danach die ED-Verbindung und das Netzmittel zugefügt.

Naßauftrag : 100 g/m²
Trockenauftrag pro m²

| | |
|---|---|
| Silberhalogenid : | 1 207 mg = 7 mMol |
| Cyanfarbstoff : | 253 mg = 0,232 mMol |
| Ascorbylpalmitat : | 497,5 mg = 1,2 mMol |
| Gelatine : | 2 939 mg |

Material II

145 g Silberchloridemulsion
155 ml destilliertes Wasser
54 g Dispergat des Blaugrünfarbstoffes
117 g Dispergat der ED-Verbindung 16
119 ml destilliertes Wasser
10 ml Netzmittel Hostapon-T (Warenzeichen) 5 %ig

Ansatz wie bei Material I
Naßauftrag : 60 g/m²
Trockenauftrag pro m²

| | |
|---|---|
| Silberhalogenid : | 1 207 mg = 7 mMol |
| Cyanfarbstoff : | 253 mg = 0,232 mMol |
| ED-Verbindung 16 : | 726 mg = 1,2 mMol |
| Gelatine | 2 422 mg |

Material III

145 g Silberchloridemulsion
155 ml destilliertes Wasser
115 g Dispergat des Magentafarbstoffes
270 g Dispergat von Ascorbylpalmitat

105 ml destilliertes Wasser
10 ml Netzmittel Hostapon-T (Warenzeichen)

Ansatz wie bei Material I
Naßauftrag : 80 g/$m^2$
Trockenauftrag pro $m^2$ :

| | | |
|---|---|---|
| Silberhalogenid : | 1 207 mg | = 7 mMol |
| Magentafarbstoff : | 216,6 mg | = 0,230 mMol |
| Ascorbylpalmitat : | 497,4 mg | = 1,2 mMol |
| Gelatine : | 2 902 mg | |

Material IV

145 g Silberchloridemulsion
155 ml destilliertes Wasser
115 g Dispergat des Magentafarbstoffes
117 g Dispergat der ED-Verbindung 16
285 ml destilliertes Wasser
10 ml Netzmittel Hostapon-T (Warenzeichen)

Ansatz wie bei Material I
Naßauftrag : 80 g/$m^2$
Trockenauftrag pro $m^2$ :

| | | |
|---|---|---|
| Silberhalogenid : | 1 207 mg | = 7 mMol |
| Magentafarbstoff : | 216,6 mg | = 0,230 mMol |
| ED-Verbindung 16 : | 726 mg | = 1,2 mMol |
| Gelatine : | 2 385 mg | |

Wie das Material II wurden auch die Materialien V-XI hergestellt, wobei anstelle der erfindungsgemäßen ED-Verbindung 16 die erfindungsgemäßen ED-Verbindungen 9, 14, 17, 19, 21, 23 und 24 in der gleichen molaren Menge eingesetzt wurden.

Wie das Material IV wurde auch das Material XII hergestellt, wobei anstelle der erfindungsgemäßen ED-Verbindung 16 die erfindungsgemäße ED-Verbindung 19 eingesetzt wurde.

Herstellung des Bildempfangsblattes

Auf einen opaken Schichtträger aus polyethylenkaschiertem Papier wurde eine Bildempfangsschicht (Beizschicht) aufgetragen mit folgender Zusammensetzung :

658 ml destilliertes Wasser
72 g Gelatine
10 ml Netzmittel FC-126 (Handelsprodukt der Fa. 3 M, Warenzeichen)
100 ml Ethanol
22 g Hexadecyl-triphenyl-phosphoniumbromid
128 ml destilliertes Wasser
10 ml Formaldehyd 4 %ig

Naßauftrag : 65 g/$m^2$
Trockenauftrag pro $m^2$ :

| | |
|---|---|
| Gelatine : | 4 680 mg |
| Beizmittel : | 1 430 mg |

Auf die Bildempfangsschicht wird eine Schutzschicht aufgetragen aus folgender Gießlösung :

943 ml destilliertes Wasser
40 g Gelatine
10 ml Netzmittel FT-248 (Warenzeichen)
7 ml Formaldehyd 4 %ig

Naßauftrag : 40 g/$m^2$
Trockenauftrag : (Gelatine) 1 600 mg/$m^2$.

Die lichtempfindlichen Materialien I-XII wurden hinter einem Stufenkeil 60 Sekunden mit einer 75 Watt-Glühlampe belichtet und zur Entwicklung in einem kommerziellen CP 38-Gerät (Warenzeichen) 1 Minute lang im Kontakt mit einem Bildempfangsblatt wie oben beschrieben, entwickelt, wobei ein Entwickler folgender Zusammensetzung verwendet wurde :

7 g NaOH
25 g $Na_3PO_4$
80 ml N-Methylpyrrolidon
20 ml 1-Phenyl-5-mercaptotetrazol (1 %ige methanolische Lösung)
5 g KBr

auffüllen mit Wasser auf 1 000 ml.

Die erhaltenen Farbkeile wurden mit einem RD 514 (Warenzeichen) Macbeth-Densitometer ausgemessen und die festgestellten Maximal- und Minimalfarbdichten sind aus der folgenden Tabelle ersichtlich.

Tabelle

| Material | ED-Verb. | Cyan Dmin | Cyan Dmax | Magenta Dmin | Magenta Dmax |
|---|---|---|---|---|---|
| I | AP | 0,01 | 0,53 | | |
| II | 16 | 0,17 | 1,99 | | |
| III | AP | | | 0,03 | 0,14 |
| IV | 16 | | | 0,10 | 0,80 |
| V | 9 | 0,02 | 0,77 | | |
| VI | 14 | 0,02 | 0,74 | | |
| VII | 17 | 0,26 | 1,82 | | |
| VIII | 19 | 0,14 | 1,85 | | |
| IX | 21 | 0,15 | 1,48 | | |
| X | 23 | 0,49 | 1,98 | | |
| XI | 24 | 0,36 | 1,88 | | |
| XII | 19 | | | 0,10 | 1,23 |

AP = Ascorbylpalmitat

**Ansprüche**

1. Farbphotographisches Aufzeichnungsmaterial mit mindestens einer lichtempfindlichen Silberhalogenidemulsionsschichteinheit und einer dieser zugeordneten Kombination aus einer nichtdiffundierenden reduzierbaren farbgebenden Verbindung, die im reduzierten Zustand unter den alkalischen Entwicklungsbedingungen einen diffundierenden Farbstoff freizusetzen vermag, und einer Elektronendonorverbindung (ED-Verbindung), die unter den alkalischen Entwicklungsbedingungen die nichtdiffundierende reduzierbare farbgebende Verbindung zu reduzieren vermag, dadurch gekennzeichnet, daß das Material als ED-Verbindung eine Verbindung der folgenden allgemeinen Formel enthält

$$R^1(-L^1=L^2)_n-NH-SO_2-X$$

worin bedeuten

$L^1$, $L^2$ je eine gegebenenfalls substituierte Methingruppe $(-\overset{|}{C}=, =\overset{|}{C}-)$, die Glied eines wenigstens partiell ungesättigten carbocyclischen oder heterocyclischen Ringsystems sein kann,

$R^1$ $-OR^2$, $-SR^2$, $-NHR^3$

$R^2$ Wasserstoff oder einen unter den Bedingungen der photographischen Entwicklung hydrolysierbaren Rest

$R^3$ Wasserstoff, Alkyl, Aryl, Acyl oder einen Rest, der zusammen mit dem in $R^1$ definierten Stickstoffatom und $L^1$ definierten Stickstoffatom und $L^1$, gegebenenfalls unter Einbeziehung von $L^2$ ein Ringsystem mit mindestens einem 5-, 6- oder 7-gliedrigen heterocyclischen Ring vervollständigt,

n 1 oder 2,

X einen beliebigen nichtfarbigen organischen Rest,

wobei mindestens $R^1$, $L^1$, $L^2$ oder ein durch die genannten Gruppen ($R^1$, $L^1$, $L^2$) vervollständigter Ring, oder X einen diffusionsfestmachenden Rest enthält.

2. Farbphotographisches Aufzeichnungsmaterial nach Anspruch 1, dadurch gekennzeichnet, daß es als ED-Verbindung eine Verbindung der folgenden allgemeinen Formel enthält

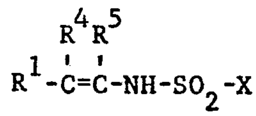

worin bedeuten

$R^1$ $—OR^2$, $—SR^2$, $—NHR^3$

$R^2$ Wasserstoff oder einen unter den Bedingungen der photographischen Entwicklung hydrolysierbaren Rest

Wasserstoff, Alkyl, Aryl, Acyl oder einen Rest, der zusammen mit $R^4$ oder mit $R^5$ oder mit $R^4$ und $R^5$ ein Ringsystem mit mindestens einem 5-, 6- oder 7-gliedrigen heterocyclischen Ring vervollständigt

$R^4$ Wasserstoff, Hydroxyl, Alkyl, Aryl, Acyl, einschließlich solcher Alkyl-, Aryl- und Acylreste, die zusammen mit wenigstens einem der Reste $R^1$ und $R^5$ ein Ringsystem mit mindestens einem 5-, 6- oder 7-gliedrigen Ring vervollständigen, oder ein Stickstoffatom mit zwei Substituenten, von denen der eine ein Wasserstoffatom ist oder ein Alkyl-, Aryl- oder Acylrest, einschließlich solcher Alkyl-, Aryl- und Acylreste, die zusammen mit $R^1$ einen 5-, 6- oder 7-gliedrigen Heteroring mit mindestens einem Stickstoffatom vervollständigen, und von denen der andere ein Wasserstoffatom ist oder ein Rest, der zusammen mit $R^5$ einen 5-, 6- oder 7-gliedrigen Heteroring mit mindestens einem Stickstoffatom vervollständigt, wobei die beiden Substituenten am Stickstoff nicht gleichzeitig Wasserstoff sein können,

$R^5$ Wasserstoff oder einen Rest, der zusammen mit $R^1$ einen 5-, 6- oder 7-gliedrigen stickstoffhaltigen heterocyclischen Ring oder zusammen mit $R^4$ einen 5-, 6- oder 7- gliedrigen carbocyclischen oder heterocyclischen Ring vervollständigt

X einen beliebigen nichtfarbigen organischen Rest,

wobei wenigstens einer der Reste $R^1$, $R^4$, $R^5$ oder ein Substituent an einem durch mindestens zwei der genannten Reste vervollständigten Ring, oder die Gruppe X einen diffusionsfestmachenden Rest enthält.

3. Farbphotographisches Aufzeichnungsmaterial nach Anspruch 1, dadurch gekennzeichnet, daß es als Ed-verbindung eine Verbindung der folgenden allgemeinen Formel enthält,

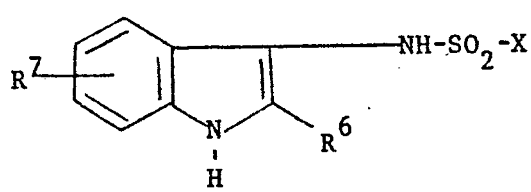

worin bedeuten

X einen beliebigen nichtfarbigen organischen Rest

$R^6$ Wasserstoff, Hydroxyl, Alkyl, Aryl, Carbamoyl oder Sulfonylamino ($—NH—SO_2—R$, wobei R = Alkyl, Aryl oder Aralkyl)

$R^7$ Wasserstoff oder Alkyl, Aryl, Aralkyl, Halogen, $NO_2$, CN, COOH, COOR, Carbamoyl, $SO_3$, Sulfamoyl, Acylamido, OH, OR, Acyloxy, wobei R = Alkyl, Aryl oder Aralkyl,

und wobei mindestens einer der Reste $R^6$, $R^7$ und X einen diffusionsfestmachenden Rest enthält.

## Claims

1. Colour photographic recording material comprising at least one light-sensitive silver halide emulsion layer unit and, associated with this unit, a combination of a non-diffusing, reducible colour providing compound which, in the reduced state, is capable of releasing a diffusible dye under alkaline development conditions, and an electron donor compound (ED compound) which is capable of reducing the non-diffusing, reducible colour providing compound under the alkaline development conditions, characterised in that the material contains, as ED compound, a compound of the following general formula

$$R^1(—L^1 = L^2)_n—NH—SO_2—X$$

in which

$L^1$, $L^2$ each represent an optionally substituted methine group ($—\overset{|}{C} =, = \overset{|}{C}—$) which may be a member

of an at least partially unsaturated carbocyclic or heterocyclic ring system,

$R^1$ represents $—OR^2$, $—SR^2$ or $—NHR^3$,

$R^2$ represents hydrogen or a group which is capable of being hydrolysed under the conditions of photographic development,

$R^3$ represents hydrogen, alkyl, aryl, acyl or a group which together with the nitrogen atom defined in $R^1$ and with $L^1$, optionally with the inclusion of $L^2$, completes a ring system having at least one 5-, 6- or 7-membered heterocyclic ring,

n represents 1 or 2, and

X represents any colourless organic group, at least $R^1$, $L^1$, $L^2$ or a ring completed by the said groups ($R^1$, $L^1$, $L^2$) or X contains a group which confers diffusion resistance.

2. Colour photographic recording material according to Claim 1, characterised in that it contains, as ED compound, a compound of the following general formula

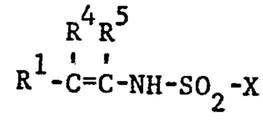

in which

$R^1$ represents $—OR^2$, $—SR^2$ or $—NHR^3$,

$R^2$ represents hydrogen or a group capable of being hydrolysed under the conditions of photographic development,

$R^3$ represents hydrogen, alkyl, aryl, acyl or a group which, together with $R^4$ or $R^5$ or with $R^4$ and $R^5$ completes a ring system having at least one 5- 6- or 7-membered heterocyclic ring,

$R^4$ represents hydrogen, hydroxyl, alkyl, aryl or acyl, including those alkyl, aryl and acyl groups which together with at least one of the groups $R^1$ and $R^5$ complete a ring system having at least one 5-, 6- or 7-membered ring, or it represents a nitrogen atom having two substituents, one of which substituents is a hydrogen atom or an alkyl aryl or acyl group, including those alkyl, aryl and acyl groups which together with $R^1$ complete a 5-, 6- or 7-membered heterocyclic ring having at least one nitrogen atom, the other substituent being a hydrogen atom or a group which together with $R^5$ completes a 5-, 6- or 7-membered heterocyclic ring having at least one nitrogen atom, but the two substituents on the nitrogen atom must not both be hydrogen,

$R^5$ represents hydrogen or a group which together with $R^1$ complete a 5-, 6- or 7-membered heterocyclic ring containing nitrogen or together with $R^4$ completes a 5-, 6- or 7-membered carbocyclic or heterocyclic ring, and

X represents any coulourless organic group,

at least one of the groups $R^1$, $R^4$, $R^5$ or a substituent on a ring completed by at least two of the aforesaid groups, or the group X containing a group which confers diffusion resistance.

3. Colour photographic recording material according to Claim 1, characterised in that it contains, as ED compound, a compound of the following general formula

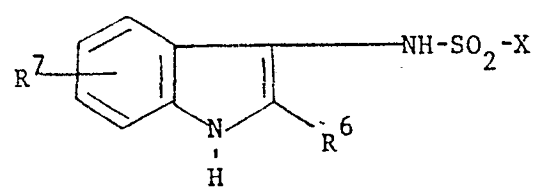

in which

X represents any coulourless organic group,

$R^6$ represents hydrogen, hydroxyl, alkyl, aryl, carbamoyl or sulphonylamino ($—NH—SO_2—R$, in which R represents alkyl, aryl or aralkyl), and

$R^7$ represents hydrogen or alkyl, aryl, aralkyl, halogen, $NO_2$, CN, COOH, COOR, carbamoyl, $SO_3$, sulphamoyl, acylamido, OH, OR or acyloxy, in which R represents alkyl, aryl or aralkyl, at least one of the groups $R^6$, $R^7$ and X containing a group which confers diffusion resistance.

**Revendications**

1. Matériau d'enregistrement photographique couleur ayant au moins une unité de couche d'émulsion d'halogénure d'argent sensible à la lumière et une combinaison associée à celle-ci d'un composé chromogène réductible non diffusible qui est capable, à l'état réduit, de libérer dans les conditions alcalines de développement un colorant diffusible et d'un composé donneur d'électrons (composé DE) qui, dans les conditions alcalines de développement, est capable de réduire le composé chromogène réductible non diffusible, caractérisé en ce que le matériau contient comme composé DE un composé de formule générale suivante

$$R^1(—L^1 = L^2)_n—NH—SO_2—X$$

dans laquelle

$L^1$, $L^2$ représentent chacun un groupe méthine éventuellement substitué (—C=,=C—) qui peut faire partie d'un système cyclique carbocyclique ou hétérocyclique au moins partiellement insaturé,

$R^1$ est un groupe $—OR^2$, $—SR^2$, $—NHR^3$,

$R^2$ représente l'hydrogène ou un reste hydrolysable dans les conditions du développement photographique,

$R^3$ représente l'hydrogène ou un groupe alkyle, aryle ou acyle, ou un reste qui, avec l'atome d'azote défini dans $R^1$ et $L^1$, éventuellement avec $L^2$, complète un système cyclique ayant au moins un noyau hétérocyclique à 5, 6 ou 7 chaînons,

n est égal à 1 ou 2,

X est un reste organique non coloré quelconque,

l'un au moins des groupes $R^1$, $L^1$ et $L^2$, ou un noyau complété par les groupes mentionnés ($R^1$, $L^1$, $L^2$) ou X contenant un reste conférant la résistance à la diffusion.

2. Matériau d'enregistrement photographique couleur selon la revendication 1, caractérisé en ce qu'il contient comme composé DE un composé de formule générale

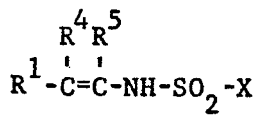

dans laquelle

$R^1$ représente $—OR^2$, $—SR^2$, $—NHR^3$,

$R^2$ représente l'hydrogène ou un reste hydrolysable dans les conditions du développement photographique,

$R^3$ représente l'hydrogène ou un groupe alkyle, aryle, ou acyle, ou un reste qui, pris avec $R^4$ ou avec $R^5$ ou avec $R^4$ et $R^5$, complète un système cyclique ayant au moins un noyau hétérocyclique à 5, 6 ou 7 chaînons,

$R^4$ représente l'hydrogène ou un groupe hydroxyle, alkyle, aryle, acyle, y compris les restes alkyle, aryle et acyle qui, pris ensemble avec au moins l'un des restes $R^1$ et $R^5$, complètent un système cyclique ayant au moins un noyau à 5, 6 ou 7 chaînons, ou bien un atome d'azote portant deux substituants dont l'un est un atome d'hydrogène, ou un reste alkyle, aryle ou acyle, y compris les restes alkyle, aryle et acyle qui, pris ensemble avec $R^1$, forment un hétérocycle à 5, 6 ou 7 chaînons ayant au moins un atome d'azote et dont l'autre est un atome d'hydrogène ou un reste qui, pris ensemble avec $R^5$, complète un hétérocycle à 5, 6 ou 7 chaînons ayant au moins un atome d'azote, et deux substituants à l'azote ne pouvant pas être en même temps l'hydrogène,

$R^5$ représente l'hydrogène ou un reste qui, pris ensemble avec $R^1$, complète un noyau hétérocycle azoté à 5, 6 ou 7 chaînons, ou pris ensemble avec $R^4$ complète un noyau carbocyclique ou hétérocyclique à 5, 6 ou 7 chaînons,

X est un reste organique non coloré quelconque,

l'un au moins des restes $R^1$, $R^4$, $R^5$ ou un substituant sur un noyau complété par au moins deux des restes mentionnés ou le groupe X contient un reste conférant la résistance à la diffusion.

3. Matériau d'enregistrement photographique couleur selon la revendication 1, caractérisé en ce qu'il contient comme composé DE un composé de formule générale suivante

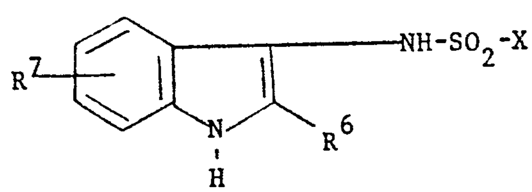

dans laquelle

X représente un reste organique non coloré quelconque,

$R^6$ représente l'hydrogène ou un groupe hydroxyle, alkyle, aryle, carbamoyle ou sulfonylamino ($—NH—SO_2—R$, où R = alkyle, aryle ou arylalkyle),

$R^7$ représente l'hydrogène ou un groupe alkyle, aryle, arylalkyle, un halogène, un groupe $NO_2$, CN, COOH, COOR, carbamoyle, $SO_3$, sulfamoyle, acylamido, OH, OR, acyloxy, où R = alkyle, aryle ou arylalkyle,

et l'un au moins des restes $R^6$, $R^7$ et X contient un reste conférant la résistance à la diffusion.